# EUROPÄISCHE PATENTSCHRIFT

(11) **EP 0 483 061 B1**
(45) Veröffentlichungstag und Bekanntmachung des Hinweises auf die Patenterteilung: **17.05.1995**
(21) Anmeldenummer: 91810794.7
(22) Anmeldetag: 15.10.1991
(51) Int. Cl.: C07D 213/643, A01N 43/40

(54) **Verfahren zur Herstellung von (3-Fluor-pyridin-2-yloxy)-phenoxy-propionsäuren**
Process for the preparation of 4-(3-fluoro-pyridine-2-yloxy)-phenoxy-propionic acids
Procédé pour la préparation d'acides 4-(3-fluoropyridine-2-yloxy)-phénoxypropionique

(30) Priorität: 23.10.1990 CH 3379/90
(43) Veröffentlichungstag der Anmeldung: 29.04.1992
(73) Patentinhaber: CIBA-GEIGY AG, 4002 Basel (CH)
(72) Erfinder: Hässig, Robert, Dr., CH-5264 Gipf-Oberfrick (CH); Siegrist, Urs, CH-5268 Eiken (CH)

(56) Entgegenhaltungen:
- EP-A- 0 305 593
- JOURNAL OF THE AMERICAN CHEMICAL SOCIETY. Bd. 72, 1950, GASTON, PA US Seiten 4809 - 4810; R.L. FERM ET AL.: 'Synthesis of aromatic fluorides through diazotation in anhydrous hydrogen fluoride'

## Beschreibung

Die vorliegende Erfindung betrifft ein Verfahren zur Herstellung von (3-Fluor-pyridin-2-yloxy)-phenoxy-propionsäuren der Formel I 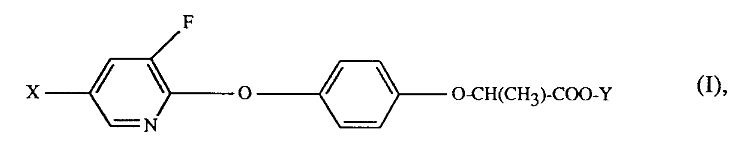
worin X für Wasserstoff, Fluor, Chlor, Brom oder Trifluormethyl und Y für Wasserstoff, Natrium oder Kalium steht.

Die (3-Fluor-pyridin-2-yloxy)-phenoxy-propionsäuren und Salze der Formel I besitzen herbizide Eigenschaften. Darüber hinaus sind sie wertvolle Zwischenprodukte zur Herstellung von herbizid wirksamen (3-Fluor-pyridin-2-yloxy)-phenoxy-propionsäureestern, wie sie beispielsweise in den veröffentlichten europäischen Patentanmeldungen EP-A-0 083 556 EP-A- 305 593 und EP-A-0 248 968 offenbart sind.

Es ist daher das Ziel der vorliegenden Erfindung, ein Verfahren bereitzustellen, welches die Herstellung von (3-Fluor-pyrindin-2-yloxy)-phenoxy-propionsäuren ausgehend von leicht zugänglichen Ausgangsstoffen auf einfache Weise und in guten Ausbeuten ermöglicht, und welches sich besonders als industrielles Produktionsverfahren eignet.

Es wurde nun gefunden, dass man (3-Fluor-pyridin-2-yloxy)-phenoxy-propionsäuren der Formel I in vorteilhafter Weise herstellen kann, wenn man eine Verbindung der Formel II 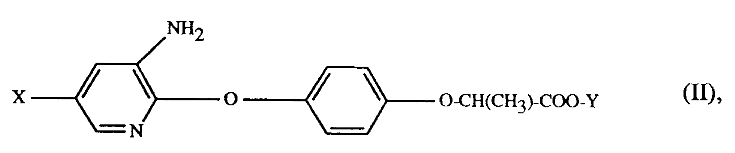
worin X und Y die unter Formel I angegebene Bedeutung haben, mit einem Diazotierungsmittel umsetzt und das gebildete Diazoniumfluorid durch thermische Zersetzung in die (3-Fluor-pyridin-2-yloxy)-phenoxy-propionsäure der Formel I überführt, wobei erfindungsgemäß die Reaktion in einem wasserfreien Medium von Fluorwasserstoff und Dimethylsulfoxid unter Normaldruck stattfindet.

Die Endprodukte des erfindungsgemäßen Verfahrens sind bekannt. Die Ausgangsprodukte der Formel II sind neu und können durch Reduktionsverfahren aus den entsprechenden Nitroverbindungen der Formel III 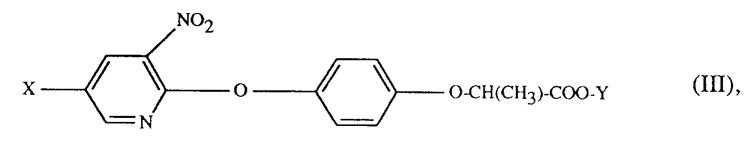
worin X und Y die unter Formel I angegebene Bedeutung haben, erhalten werden. Zur Reduktion der Nitroverbindungen der Formel III zu den Aminoverbindungen der Formel II sind alle gängigen in der Literatur beschriebenen Verfahren einsetzbar. Beispielsweise läßt sich die Reduktion mit Vorteil in wäßrigem Medium in Gegenwart von Eisen, Zinn oder Zink und Salzsäure durchführen. Weitere geeignete Methoden sind Reduktionsverfahren unter Verwendung komplexer Hydride wie Lithiumaluminiumhydrid oder die katalytische Reduktion mit Wasserstoff unter Zuhilfenahme von Platin-, Palladium- oder Nikkelkatalysatoren.

Die Verbindungen der Formel III, die als Ausgangsprodukte zur Herstellung der Verbindungen der Formel II eingesetzt werden, sind ebenfalls neu und können nach an sich bekannten Methoden hergestellt werden, beispielsweise durch Umsetzung der entsprechenden 2-Chlor-3-nitropyridine mit 2-(4-Hydroxy)-phenoxypropionsäure in Gegenwart einer Base. Derartige Verfahren sind beispielsweise in der EP-A-0 248 968 beschrieben. Die zur Herstellung der Verbindungen der Formel III erforderlichen 2-Chlor-3-nitropyridine sowie die 2-(4-Hydroxy)-phenoxypropionsäure sind bekannt oder nach bekannten, dem Fachmann geläufigen Methoden herstellbar.

Die Diazotierung kann bei Temperaturen von -20 bis +25°C durchgeführt werden, bevorzugt ist ein Temperaturbereich von -10 bis + 10°C.

Bevorzugte Diazotierungsmittel sind Natriumnitrit, Kaliumnitrit, N₂O₃, NOF, NOCl, iso-Amylnitrit oder tert.-Butylnitrit. Ein ganz besonder bevorzugtes Diazotierungsmittel ist Natriumnitrit.

Die thermische Zersetzung des Diazoniumfluorids der Formel IV, 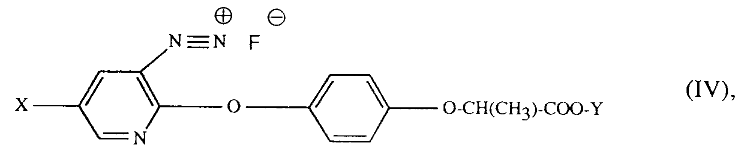
worin X und Y die unter Formel I angegebene Bedeutung haben, wird im allgemeinen bei Temperaturen von +15 bis + 100°C, vorzugsweise bei Temperaturen von +30 bis + 60°C durchgeführt. Ganz besonders bevorzugt ist ein Temperaturbereich von +45 bis +60°C.

Ein geeignetes wasserfreies Medium ist ein Gemisch aus Fluorwasserstoff und Dimethylsulfoxid.

Erfindungsgemäß legt man Fluorwasserstoff und die Verbindung der Formel II im wasserfreien Medium vor und gibt anschliessend das Diazotierungsmittel zu.

Das erfindungsgemäße Verfahren kann sowohl in 2 Schritten als auch in einem Schritt durchgeführt werden. Bei der zweistufigen Variante des erfindungsgemäßen Verfahrens wird in der ersten Stufe das Diazoniumfluorid hergestellt und dieses anschließend in der zweiten Stufe thermisch zersetzt. Bei der besonders bevorzugten einstufigen Variante des erfindungsgemäßen Verfahrens erfolgt die Diazotierung bei der Zersetzungstemperatur des in situ entstehenden Diazoniumfluorids, welches unmittelbar in die Verbindung der Formel I überführt wird.

In einer ganz besonders bevorzugten Variante des erfindungsgemäßen Verfahrens setzt man ein Gemisch aus Fluorwasserstoff und 4-(5-Chlor-3-amino-pyridin-2-yloxy)-phenoxy-propionsäure in Gegenwart von Dimethylsulfoxid bei einer Temperatur von +45 bis +60°C mit Natriumnitrit um.

Mit dem erfindungsgemäßen Verfahren lassen sich die (3-Fluor-pyridin-2-yloxy)-phenoxy-propionsäuren der Formel I aus leicht zugänglichen Ausgangsprodukten auf einfache Weise und in guten Ausbeuten und hoher Selektivität und Reinheit herstellen. Ein besonderer Vorteil des erfindungsgemäßen Verfahrens besteht darin, daß die unbeständigen und explosiven Diazoniumsalz-Zwischenprodukte nicht aus dem Reaktionsmedium isoliert werden müssen. Ferner entstehen keinerlei ökologisch problematische Fluorid- oder Bortrifluoridabfälle, da der überschüssige Fluorwasserstoff auf einfache Weise zurückgeführt werden kann.

Das erfindungsgemäße Verfahren wird durch die folgenden Beispiele näher erläutert.

### Herstellungsbeispiele:

### A) Herstellung der Ausgangsprodukte

### Beispiel A1: Herstellung von 5-Chlor-2-aminopyridin:

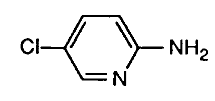

Zu 4 l konzentrierter Salzsäure gibt man bei einer Temperatur von +10°C innerhalb von 30 Minuten 941 g 2-Aminopyridin hinzu. Anschließend leitet man innerhalb von 8 1/2 Stunden bei einer Temperatur von +10°C 745 g gasförmiges Chlor ein. Nach 18-stündigem Rühren neutralisiert man das Reaktionsgemisch bei einer Temperatur von +10 bis +20°C mit 5,9 l konzentrierter Natronlauge bis zu einem pH von 7 bis 8. Das ausgefallene Rohprodukt wird filtriert, mit Eiswasser gewaschen und anschließend bei einer Temperatur von +40°C getrocknet. Zur Reinigung wird das Rohprodukt in Wasser aufgeschlemmt und anschließend filtriert. Nach Aufschlämmen in Petrolether, abfiltrieren und Trocknen des Produkts erhält man 991 g (77,1% d.Th.) 5-Chlor-2-aminopyridin mit einem Schmelzpunkt von +128 bis +130°C und einem Gehalt von 95%.

### Beispiel A2: Herstellung von 5-Chlor-2-hydroxy-3-nitropyridin:

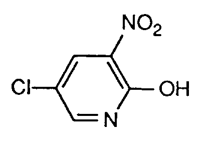

Zu 1,25 l konzentrierter Schwefelsäure gibt man unter Kühlung 321 g 5-Chlor-2-aminopyridin tropfenweise hinzu. Anschließend rührt man die Reaktionsmischung bis zur vollständigen Losung des Eduktes. Nun gibt man bei einer Temperatur von +40 bis +45°C 172.5 g in 240 ml Wasser gelöstes Natriumnitrit hinzu und rührt die Reaktionsmischung 15 Minuten lang. Anschließend gibt man bei einer Temperatur von +50°C innerhalb von 40 Minuten 125 ml Salpetersäure (100%) tropfenweise hinzu. Nun wird die Reaktionsmischung 1 Stunde lang bei einer Temperatur von +55°C gehalten und anschließend in 5kg Eiswasser gegeben. Das als gelber Niederschlag ausfallende Produkt wird filtriert, mit viel Wasser gewaschen und anschließend bei einer Temperatur von +60°C getrocknet. Man erhält 351 g 5-Chlor-2-hydroxy-3-nitropyridin (80,5% d.Th) mit einem Schmelzpunkt von +229 bis +231°C.

### Beispiel A3: Herstellung von 2,5-Dichlor-3-nitropyridin:

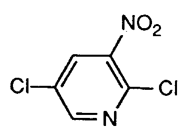

Zu einer Mischung von 87,5 g 5-Chlor-2-hydroxy-3-nitropyridin in 500 ml Toluol und 3 ml N,N-Dimethylformamid leitet man bei einer Temperatur von +85 bis +95°C innerhalb 1 Stunde 75 g Phosgen ein. Nach 2-stündigem Halten der Reaktionsmischung unter Rückfluß bei einer Temperatur von +85 bis +95°C laßt man über einen Zeitraum von 18 Stunden auf Raumtemperatur abkühlen. Nach 3-maligem Waschen der Reaktionsmischung mit Wasser wird die organische Phase mit Natriumsulfat getrocknet und anschließend eingeengt. Man erhält Rohprodukt in Form eines braunen Öles, welches aus 200 ml n-Hexan kristallisiert wird. Man erhält 67,5 g 2,5-Dichlor-3-nitropyridin (70% d.Th.) mit einem Schmelzpunkt von +38 bis +39°C.

### Beispiel A4: Herstellung von 2-(4-(5-Chlor-3-nitropyridin-2-yloxy)-phenoxy)-propionsäure:

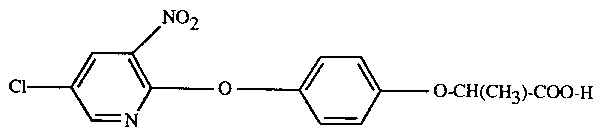

Zu einer Suspension aus 91 g Wasser und 92,3 g 2-(4-Hydroxy)-phenoxypropionsäure gibt man bei Raumtemperatur 56,1 g 50%-ige wäßrige Kaliumhydroxidlösung tropfenweise hinzu. Nach Entfernen des Wassers durch Destillation löst man das entstandene Kaliumsalz in 390 g N,N-Dimethylformamid und gibt anschließend bei einer Temperatur von +85°C 108,1 g 2,5-Dichlor-3-nitropyridin in 95 g N,N-Dimethylformamid tropfenweise hinzu. Nach Rühren der Reaktionsmischung für 90 Minuten bei einer Temperatur von +85°C gibt man das Gemisch in Wasser und läßt 100 ml konzentrierte Salzsäure zutropfen. Das in öliger Form ausfallende Rohprodukt wird mit Toluol extrahiert. Nach dem Einengen der organischen Phase erhält man 188 g (92% d.Th.) 2-(4-(5-Chlor-3-nitropyridin-2-yloxy)-phenoxy)-propionsäure.

### Beispiel A5: Herstellung von 2-(4-(5-Chlor-3-aminopyridin-2-yloxy)-phenoxy)-propionsäure:

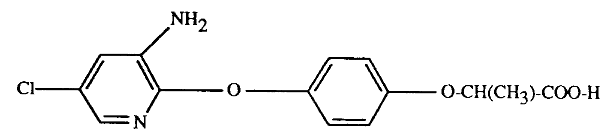

Eine Losung von 196 g 2-(4-(5-Chlor-3-nitropyridin-2-yloxy)-phenoxy)-propionsäure in 615 g Dioxan versetzt man mit 20 g Raney-Nickel-Katalysator und hydriert anschließend bei einer Temperatur von +20 bis +22°C unter Normaldruck. Nach Abtrennen des Katalysators wird das Losungsmittel entfernt. Man erhält 98 g 2-(4-(5-Chlor-3-aminopyridin-2-yloxy)-phenoxy)-propionsäure als Rohprodukt (55% d.Th.) mit einem Schmelzpunkt von +152 bis +154°C, welches aus Toluol umkristallisiert werden kann.

### B) Herstellung der Endprodukte

### Beispiel B1: Herstellung von 2-(4-(5-Chlor-3-fluorpyridin-2-yloxy)-phenoxy)-propionsäure:

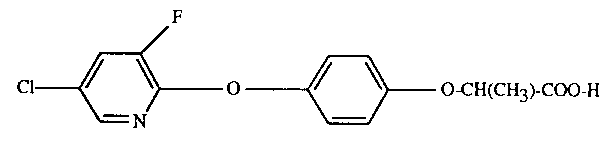

In einem Polyethylenreaktor kondensiert man bei einer Temperatur von -78°C 150 g Fluorwasserstoff ein. Anschließend gibt man innerhalb 1 Stunde bei einer Temperatur von -20 bis +10°C eine Losung aus 31,2 g 2-(4-(5-Chlor-3-aminopyridin-2-yloxy)-phenoxy)-propionsäure in 82,5 g Dimethylsulfoxid tropfenweise hinzu. Nach langsamem Erwärmen der Reaktionsmischung auf eine Temperatur von +50 °C gibt man bei einer maximalen Temperatur von +60 °C portionsweise über einen Zeitraum von 90 Minuten 7.4 g Natriumnitrit hinzu. Anschließend wird das Reaktionsgemisch auf Raumtemperatur gekühlt und mit Essigsäureethylester versetzt. Nach Einrühren der Reaktionsmischung in Eis neutralisiert man mit konzentriertem Ammoniak bis zu einem pH von 3 bis 4. Nach mehrfacher Extraktion mit Essigsäureethylester werden die vereinigten organischen Phasen mit Wasser bis zu einem pH von 8 extrahiert. Anschließend wird die wäßrige Phase mit konzentrierter Salzsäure auf einen pH kleiner als 2 eingestellt. Nach Entfernen des Losungsmittels erhält man 30 g 2-(4-(5-Chlor-3-fluorpyridin-2-yloxy)-phenoxy)-propionsäure (88% d.Th.) mit einem Gehalt von 90 bis 95% und einem Schmelzpunkt von +90 bis +92°C, welches aus Methanol/Wasser oder Toluol umkristallisiert werden kann.

## Patentansprüche

1. Verfahren zur Herstellung von (3-Fluor-pyridin-2-yloxy)-phenoxy-propionsäuren der Formel I 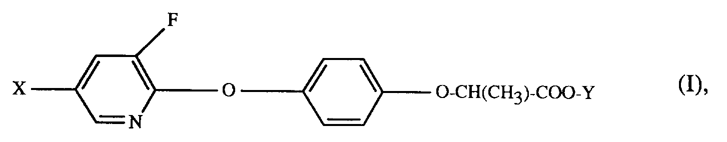 worin X für Wasserstoff, Fluor, Chlor, Brom oder Trifluormethyl und Y für Wasserstoff, Natrium oder Kalium steht,
durch Umsetzung einer Verbindung der Formel II 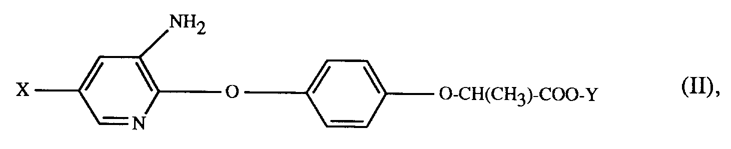 worin X und Y die unter Formel I angegebene Bedeutung haben, mit einem Diazotierungsmittel und thermischer Zersetzung des gebildeten Diazoniumfluorid in die (3-Fluor-pyridin-2-yloxy)-phenoxy-propionsäure der Formel I oder deren Natrium- oder Kaliumsalze,
dadurch gekennzeichnet, dass die Reaktion in einem wasserfreien Medium von Fluorwasserstoff und Dimethylsulfoxid unter Normaldruck stattfindet.

2. Verfahren nach Anspruch 1, dadurch gekennzeichnet, daß man die thermische Zersetzung bei einer Temperatur von +30 bis +60°C durchführt.

3. Verfahren nach Anspruch 1, dadurch gekennzeichnet, daß man die thermische Zersetzung bei einer Temperatur von +45 bis +60°C durchführt.

4. Verfahren nach Anspruch 1, dadurch gekennzeichnet, daß man als Diazotierungsmittel Natriumnitrit, Kaliumnitrit, N₂O₃, NOF, NOCl, iso-Amylnitrit oder tert.-Butylnitrit verwendet.

5. Verfahren nach Anspruch 1, dadurch gekennzeichnet, daß man als Diazotierungsmittel Natriumnitrit verwendet.

6. Verfahren nach Anspruch 1, dadurch gekennzeichnet, daß man Fluorwasserstoff, Dimethylsulfoxid und die Verbindung der Formel II im wasserfreien Medium vorlegt und anschliessend das Diazotierungsmittel zugibt.

## Claims

1. A process for the preparation of a (3-fluoropyridin-2-yloxy)phenoxypropionic acid of formula I 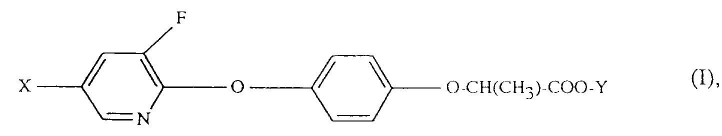 wherein X is hydrogen, fluoro, chloro, bromo or trifluoromethyl and Y is hydrogen, sodium or potassium,
by reacting a compound of formula II 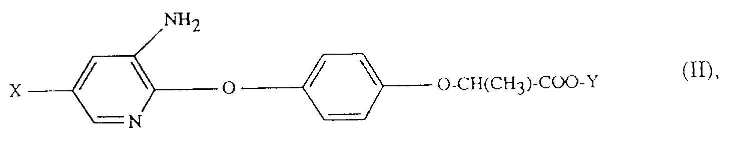 wherein X and Y are as defined for formula I, with a diazotising agent, and converting the resulting diazonium fluoride by thermal decomposition into the (3-fluoropyridin-2-yloxy)-phenoxypropionic acid of formula I or the sodium or potassium salt thereof, wherein the reaction takes place in an anhydrous medium of hydrogen fluoride and dimethyl sulfoxide under normal pressure.

2. A process according to claim 1, wherein the thermal decomposition is carried out at a temperature from +30 to +60°C.

3. A process according to claim 1, wherein the thermal decomposition is carried out at a temperature from +45 to +60°C.

4. A process according to claim 1, wherein sodium nitrite, potassium nitrite, N₂O₃, NOF, NOCl, isoamyl nitrite or tert-butyl nitrite is used as the diazotising agent.

5. A process according to claim 1, wherein sodium nitrite is used as the diazotising agent.

6. A process according to claim 1, which comprises adding the diazotising agent to hydrogen fluoride, dimethyl sulfoxide and the compound of formula II in the anhydrous medium.

## Revendications

1. Procédé de préparation des acides (3-fluoropyridine-2-yloxy)-phénoxypropioniques de formule I 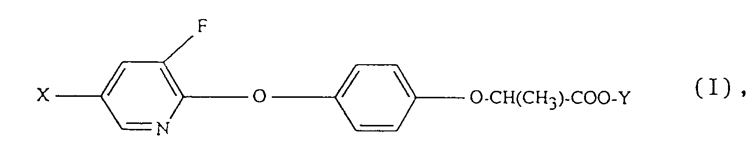 dans laquelle X représente l'hydrogène, le fluor, le chlore, le brome ou un groupe trifluorométhyle et Y représente l'hydrogène, le sodium ou le potassium,
par réaction d'un composé de formule II 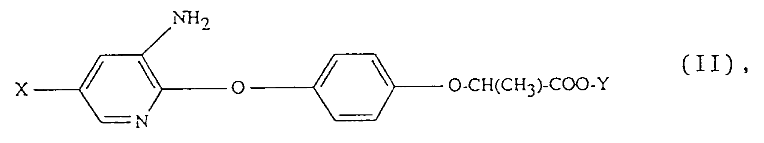 dans laquelle X et Y ont les significations indiquées en référence à la formule I, avec un agent diazotant, ce qui donne un fluorure de diazonium qu'on décompose à la chaleur en l'acide (3-fluoropyridine-2-yloxy)-phénoxypropionique de formule I ou son sel de sodium ou de potassium,
caractérisé en ce que la réaction est effectuée dans un milieu anhydre consistant en fluorure d'hydrogène et diméthylsulfoxyde, à pression normale.

2. Procédé selon revendication 1, caractérisé en ce que la décomposition à la chaleur est réalisée à une température de +30 à +60°C.

3. Procédé selon revendication 1, caractérisé en ce que la décomposition à la chaleur est réalisée à une température de +45 à +60°C.

4. Procédé selon revendication 1, caractérisé en ce que l'on utilise en tant qu'agent diazotant le nitrite de sodium, le nitrite de potassium, N₂O₃, NOF, COCl, le nitrite d'isoamyle ou le nitrite de tert-butyle.

5. Procédé selon revendication 1, caractérisé en ce que l'on utilise en tant qu'agent diazotant le nitrite de sodium.

6. Procédé selon revendication 1, caractérisé en ce que l'on ajoute l'agent diazotant à un mélange anhydre du fluorure d'hydrogène , du diméthylsulfoxyde et du composé de formule II.
